# EUROPEAN PATENT SPECIFICATION

(11) **EP 2 528 589 B1**
(45) Date of publication and mention of the grant of the patent: **16.08.2017**
(21) Application number: 11706650.6
(22) Date of filing: 31.01.2011
(51) Int. Cl.: A61K 9/16, A61K 31/545, A61K 9/20, A61P 11/00, A61P 13/00, A61P 31/00

(54) **STABLE EFERVESCENT FORMULATIONS COMPRISING CEFACLOR**
STABILE BRAUSEFORMULIERUNG ENTHALTEND CEFACLOR
FORMULATIONS EFFERVESCENTES STABLES CONTENANT DU CÉFACLOR

(30) Priority: 29.01.2010 TR 201000688
(43) Date of publication of application: 05.12.2012
(73) Proprietor: Bilgic, Mahmut, 34220 Esenler-Istanbul (TR)
(72) Inventor: Bilgic, Mahmut, 34220 Esenler-Istanbul (TR)
(86) International application number: PCT/TR2011/000037
(87) International publication number: WO 2011/093832

(56) References cited:
- EP-A1- 0 862 915
- WO-A1-99/49868
- WO-A1-2007/086012
- CN-C- 100 417 383

## Description

Present invention relates to effervescent formulations comprising cefaclor and process for their preparation.

### Background of the invention

Cefaclor is a semi-synthetic, wide spectrum, second generation cephalosporin antibiotic which is shown with Formula (I) and which is disclosed in US3925372 (A) for the first time by Eli Lilly.

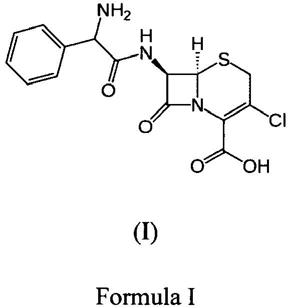

Cefaclor, which is physically in white or slightly yellow powder form, dissolve in water slightly but does not dissolve in methanol and methylene chloride. The product sold in the market under the tradename CECLOR^{®} is present in 250 and 500 mg capsule and 125 and 250 mg suspension forms.

Cefaclor is used for treatment of infections caused by gram positive microorganisms; staphylococcus aureus, Staphylococcus epidermidis, Staphylococcus saprophyticus, Staphylococcus pneumonia, Streptococcus pyogenes (group A streptococci), gram negative microorganisms; Haemophilus parainfluenzea, Haemophilus influenzae, Moraxella (Branhamella) catarrhalis, Escherichia coli, Klebsiella pneumonia, respiratory tract infections caused by Proteus mirabilisin such as (pneumonia, bronchitis, acute inflammations of chronic bronchitis, pharangitis, tonsilitis), middle-ear infections, sinusitis, skin and soft tissue infections and urinary tract infections (cystitis and pyelonephritis).

Formulations comprising cefaclor are commonly present in the forms of tablet, oral suspension and capsul. The activity of cefaclor increases when cefaclor is used in the dosages of more than 500 mg. To benefit from the active agent with a maximum efficiency, the use of cefaclor in 500 mg and more than 500 mg of dosages is preferred. In the preferred dosage forms comprising 500 mg and more, large amounts of antibiotic become bigger in size when formulated with the excipients and this makes the use of drug difficult for pediatric and geriatric patients. Alternatively, the use of reliable and user-friendly effervescent forms is suggested.

Generally, the most important matters for the patient in drug usage are the suitability of drug for use, its bioavailability and long shelf life. The use of the drugs formulated in effervescent form is reliable and easy. However, it is inevitable that the drug is exposed to the possible chemical, physical and microbial reactions and/or the active agents interact with the other excipients during the process of converting the effervescent formulations into the final product and their storage. Moreover, chemical degradation arising from possible chemical reactions between active agent and excipients such as oxidation and hydrolsis and/or physical degradation arising from some factors such as pH, temperature, light, carbondioxide and moisture lead to obtaining the drugs which are not suitable for use. During the use or storage of the active agent, these unstable drugs cause the loss or inactivation of the active agent; conversion of it into undesirable forms or failure in complete dispersion in water or failure in homogenous dispersion even though it does disperse; loss of content uniformity and therefore, a decrease in the bioavailability of the medicament.

As is seen, there is a need for development of new effervescent formulations in order to obtain stable drugs that do not degrade easily, have long shelf-life, have high bioavailability and are suitable for use.

Inventors have surprisingly seen that efferescent formulations comprising cefaclor prepared according to present invention solve the problems in the prior art.

### Description of the Invention

The present invention is related to effervescent formulations comprising cefaclor active agent and process for the preparation of these formulations. Surprisingly, when pharmaceutically acceptable amounts of cefaclor in hydrate form, at least one acid anhydrous and carbonate anhydrous are used, effervescent formulations which, do not undergo chemical or physical degradation, do not have problems by means of content uniformity or active agent loss, can disperse in water homogeneously and have high bioavailability, thus are highly stable are obtained.

Another aspect of the present invention is that the effervescent formulations comprise cefaclor in an amount of more than 500mg, preferably in the range of 500 mg- 1500 mg or its pharmaceutically acceptable derivative in an equivalent amount of that per unit dose. Accordingly, the first aspect of the present invention is that effervescent formulations comprise pharmaceutically acceptable amounts of cefaclor in hydrate form, at least one acid anhydrous and carbonate anhydrous.

Acid anhydrous which is used as effervescent acid in the effervescent formulation in accordance with the present invention can be selected from anhydrous organic acids such as citric acid anhydrous, tartaric acid anhydrous, malic acid anhydrous, fumaric acid anhydrous, ascorbic acid anhydrous, adipic acid anhydrous and succinic acid anhydrous. Preferably citric acid anhydrous is used.

In the effervescent formulation in accordance with the present invention, a second acidic agent can be used in addition to citric acid anhydrous used as effervescent acid.

Second acidic agent is selected from a group comprising sodium citrate, sodium acetate, dibasic sodium phosphate, tribasic sodium phosphate, monobasic sodium phosphate, sodium acid pyrophosphate and sodium acid sulphite.

In the effervescent formulation in accordance with the present invention, carbonate anhydrous used as effervescent base can be selected from anhydrous basic agents such as sodium carbonate anhydrous, sodium bicarbonate anhydrous, potassium carbonate anhydrous, lysine carbonate anhydrous, arginine carbonate anhydrous and calcium carbonate anhydrous. Preferably sodium bicarbonate anhydrous is used.

Cefaclor that is used in the present invention can be present in one of the forms of monohydrate, dihydrate, trihydrate and/or anhydrous. Preferably, it is used in monohydrate form.

In another aspect, the present invention is related to effervescent formulations comprising cefaclor monohydrate as active agent, citric acid anhydrous and sodium carbonate anhydrous as effervescent couple.

Generally, non-homogeneous solutions are obtained when effervescent formulations can not disperse completely upon releasing into water and/or the active agent in the formulation does not dissolve completely. This case results in a decrease in the cell absorption of active agent of the formulation and thus decrease in its bioavailability.

As a result of the studies relative to the present invention, the formulations comprising cefaclor: acid anhydrous ratio in the range of 1:10 to 8:10, preferably 2:10 to 7:10 and cefaclor: carbonate anhydrous ratio in the range of 1:10 to 9:10, preferably 1:10 to 8:10 can disperse in water easily and a homogeneous and smooth solution is obtained via the complete dissolution of the active agent present in this formulation.

In this aspect, the present invention is related to effervescent formulations comprising cefaclor: acid anhydrous ratio in the range of 1:10 to 8:10, preferably 2:10 to 7:10 and cefaclor: carbonate anhydrous ratio in the range of 1:10 to 9:10, preferably 1:10 to 8:10.

Another aspect of the present invention is that the effervescent formulation comprises cefaclor in an amount in the range of 1-60%, preferably 5-50% and more preferably 10-45% compared to the total dosage weight.

Another aspect of the present invention is pharmaceutical composition comprising cefaclor as active agent, at least one effervescent acid, at least one effervescent base and also pharmaceutically acceptable excipients.

Pharmaceutical composition formulated in the effervescent form in accordance with the present invention comprises cefaclor as active agent, at least one effervescent acid, at least one effervescent base and additionally one or more of pharmaceutically acceptable agents such as binder, lubricant, sweetener and/or taste regulator, flavoring agent, glidant, diluents, disintegrant, coloring agent, surfactant, anti-foaming agent, humectants, acidic agent and basic agent.

Binder used in the effervescent formulation in accordance with the present invention can be selected from, but not limited with, a group comprising; alginic acid, chitosan, carbomer, carboxymethyl cellulose sodium, dextrin, hydroxyethyl cellulose, hydroxymethyl cellulose, hydroxypropyl cellulose ethyl cellulose, gelatine, hypromellose, magnesium aluminium silicate, maltodextrin, polyethylene oxide and povidone or a combination thereof. Preferably povidone is used.

Lubricant used in the effervescent formulation in accordance with the present invention can be selected from, but not limited with, a group comprising PEG 6000 or sodium benzoate. Preferably, PEG 6000 is used.

Sweetener and/or taste regulator that can be used in effervescent formulations of the present invention can be selected from, but not limited with, a group of acesulfame, aspartame, dextrose, fructose, saccharine, sucralose, sucrose, , saccharin sodium, lactitol, maltitol, maltose, sorbitol, sodium cyclamate, sucrose and xylitol or a combination thereof.

Flavoring agent that can be used in effervescent formulations of the present invention can be selected from, but not limited with, natural aroma oils (peppermint oil, oil of wintergreen, oil of cloves, parsley oil, eucalyptus oil, lemon oil, orange oil), menthol, menthane, anethole, methyl salicylate, eucalyptole, cinnamon, 1-methyl acetate, sage, eugenol, oxanon, alpha-irison, marjoram, lemon, orange, blackberry, propenyl guaethol acethyl, cinnamon, vanilla, thymol, linalol, cinnamalaldehyde glycerol acethal, N-quadric p-menthan-3-carboxamide, 3,1-methoxy propane 1,2-diol or a combination thereof.

Glidant that can be used in effervescent formulations of the present invention can be selected from, but not limited with, sodium lauryl sulfate, sodium benzoate, sodium chloride, sodium acetate, sodium acetate, sodium fumarate, carbowax 4000, L-leucine(17), PEG or a combination thereof.

Diluent that can be used in effervescent formulations of the present invention can be selected from, but not limited with, a group comprising calcium carbonate, calcium sulfate, dibasic calcium phosphate, tribasic calcium phosphate, calcium sulfate, microcrystalline cellulose, lactose, magnesium carbonate, magnesium oxide, maltodextrin, maltose, mannitol, sodium chloride, sorbitol, starch and xylitol or combinations thereof.

Disintegrant that can be used in effervescent formulations of the present invention can be selected from, but not limited with, a group comprising carboxymethyl cellulose calcium, carboxymethyl cellulose sodium, microcrystalline cellulose, silicone dioxide, croscarmellose sodium, crospovidone, hydroxypropyl cellulose, methyl cellulose, povidone, magnesium aluminium silicate and starch or a combination thereof.

Coloring agent that can be used in effervescent formulations of the present invention can be selected from, but not limited with, caratenoids and chlorophyl and a combination thereof.

Surfactant that can be used in effervescent formulations of the present invention can be selected from, but not limited with, sodium lauryl sulfate, magnesium lauryl sulfate, sodium sulfate anhydrous or a combination thereof. Preferably, sodium sulfate anhydrous is used.

Anti-foaming agent that can be used in effervescent formulations of the present invention can be selected from, but not limited with, simethicone and dimethyl siloxane, silicone oil or a combination thereof.

Humectant that can be used in effervescent formulations of the present invention can be selected from, but not limited with, anhydrous sodium sulphate, silica gel and potassium carbonate or combinations thereof.

Acidic agent that can be used in effervescent formulations of the present invention can be selected from, but not limited with, a group comprising acetic acid, citric acid, lactic acid, malic acid, phosphoric acid, propionic acid, tartaric acid or combinations thereof.

Basic agent that can be used in effervescent formulations of the present invention can be selected from, but not limited with, a group comprising potassium carbonate, potassium bicarbonate, potassium citrate, potassium hydroxide, sodium carbonate, sodium bicarbonate, or combinations thereof.

"Effervescent couple" term means the use of acidic agent and basic agent together.

"Effervescent formulations" term comprises effervescent tablets, effervescent granules and effervescent powder. Effervescent formulations comprising cefaclor in accordance with the present invention is preferably compressed into tablets.

"Final product" term means that the final state of effervescent tablet, effervescent granule or effervescent powder which is obtained by producing effervescent formulations in a dosage form and ready for use.

In the effervescent pharmaceutical composition according to the present invention, 1-60% of cefaclor, 5-70 % of acid anhydrous, 5-40 % of carbonate anhydrous, 1-20 % of 0 %, surfactant, , 0.3-6 % of binder, 2-10 % of %, lubricant, 0.5-7% of sweetener and/or taste regulator, 1-4 % of %, flavoring agent % and 0-3% of coloring agent with respect to the total weight of the unit dosage can be used.

In the effervescent cefaclor formulation in accordance with the present invention, optionally a second active agent can be used. The second active agent can be selected from beta lactamase inhibitors and cephalasporins, preferably clavulanic acid or combinations thereof.

Clavulanic acid used in the effervescent cefaclor formulation in accordance with the present invention, can be present in the form of its solvates, hydrates, enetiomers, racemates, organic salts, inorganic salts, polymorphs, crystal and amorphous forms or in free form and/or as a combination of these. Preferably, potassium clavulanate is used.

Clavulanic acid and/or derivatives thereof (for example potasium clavulanate) is very sensitive to moisture. Therefore, in the pharmaceutical composition according to the present invention, potasium clavulanate is preferably used together with a humidity absorbing agent in a ratio of 1:1.

One or more than one of the following substances can be used as a humidity absorbing agent; silica; colloid silica, for instance colloidal silica anhydrous, magnesium trisilicate, powdered cellulose, magnesium oxide, calcium silicate, Syloid®, starch, microcrystalline cellulose and talc.

In the effervescent cefaclor formulation in accordance with the present invention, potasium clavulanate is preferably used with syloid or microcrystalline cellulose in an amount in the ratio of 1:1.

In the effervescent formulation comprising cefaclor in accordance with the present invention, with respect to the total weight of the unit dosage; 5-80%, preferably 10-70% of clavulanic acid or pharmaceutically acceptable salts, hydrates, solvates or a combination thereof in an amount equivalent to that can be used.

A process for the preparation of effervescent formulations comprising cefaclor in accordance with the present invention comprises the steps of;
1. Obtaining a granulation solution by mixing at least one binder, at least one lubricant and deionized water
2. Sieving effervescent acid, effervescent base and surfactant and granulating them with the granulation solution
3. Adding cefaclor, sweetener and flavoring agent after drying and sieving the granules obtained in the second step. The obtained effervescent formulation is optionally compressed into tablets in the tablet pressing machine.

In cases where effervescent formulations comprising cefaclor comprise a second active agent, for example potassium clavulanate, the process used for the preparation of said formulation comprises the steps of;
1. Blending effervescent acid, effervescent base, sweetener and binder and granulating the mixture with water and then drying and sieving the obtained granules,
2. Adding lubricant, potassium clavulanate:humidity absorbing agent (1:1) mixture, cefaclor, coloring agent and flavoring agent into the obtained granules and blending them. The obtained effervescent formulation is optionally compressed into tablets in the tablet pressing machine.

Another aspect of the present invention is that the pharmaceutical composition prepared according to the invention is used for the treatment of the diseases related to the upper respiratory tract infections such as pharangitis, tonsillitis, otitis media; lower respiratory tract infections such as acute pneumonia, acute and chronic broncihia and urinary tract infections such as acute cystitis and cystourethritis.

Though not limited with these examples, effervescent formulations according to present invention can be prepared according to the examples given below.

### Example 1: Formulation and process for preparation of effervescent tablet formulation

A granulation solution comprising binder, lubricant and deionized water is prepared. Effervescent acid, base and surfactant are granulated with the granulation solution by adding them into fluidized bed dryer. Cefaclor , sweetener and flavoring agent are added into the obtained mixture and this mixture is dried. Finally, the dried mixture is compressed into tablets.

| Component name | % amount in unit dose |
|---|---|
| Cefaclor | 15 % |
| Effervescent acid | 35 % |
| Effervescent base | 25.5 % |
| Surfactant | 18 % |
| Binder | 0.5 % |
| Sweetener | 0.75 % |
| Lubricant | 2.5 % |
| Flavoring Agent | 2.75 % |

### Example 2: Formulation and process for preparation of effervescent tablet

The mixture comprising effervescent acid, effervescent base, sweetener and binder is granulated with water in the fluidized bed dryer. The obtained granules are dried and sieved. Lubricant, potassium clavulanate: humidity absorbing agent (1:1) mixture, cefaclor, coloring agent and flavoring agent are added into the obtained granules and blended. The obtained final mixture is compressed into tablets.

| Component name | % amount in unit dose |
|---|---|
| Cefaclor | 13 % |
| potassium clavulanate:humidity absorbing agent (1:1) mixture | 8 % |
| Effervescent acid | 36 % |
| Effervescent base | 34 % |
| Coloring agent | 2.5 % |
| Binder | 2 % |
| Sweetener | 2 % |
| Lubricant | 1 % |
| Flavoring Agent | 1.5 % |

## Claims

1. An effervescent formulation **characterized in that** said composition comprises pharmaceutically acceptable amounts of cefaclor in hydrate form, at least one acid anhydrous and carbonate anhydrous.

2. An effervescent formulation according to claim 1, wherein cefaclor in hydrate form is present in the unit dose in the range of 500 mg to 1500 mg.

3. An effervescent formulation according to claims 1-2, wherein acid anhydrous which is used as effervescent acid is selected from anhydrous organic acids such as citric acid anhydrous, tartaric acid anhydrous, malic acid anhydrous, fumaric acid anhydrous, ascorbic acid anhydrous, adipic acid anhydrous and succinic acid anhydrous.

4. An effervescent formulation composition according to claim 3, wherein citric acid anhydrous is used as acid anhydrous.

5. An effervescent formulation composition according to claims 1-4, wherein carbonate anhydrous used as effervescent base is selected from anhydrous basic agents such as sodium carbonate anhydrous, sodium bicarbonate anhydrous, potassium carbonate anhydrous, lysine carbonate anhydrous, arginine carbonate anhydrous and calcium carbonate anhydrous.

6. An effervescent formulation composition according to claim 5, wherein sodium bicarbonate anhydrous is used as carbonate anhydrous.

7. An effervescent formulation composition according to claims 1-6, wherein cefaclor that is used as active agent is present in one of the forms of monohydrate, dihydrate, trihydrate.

8. An effervescent formulation composition according to claim 7, wherein cefaclor that is used as active agent is in monohydrate form.

9. An effervescent formulation composition according to claims 1-8, wherein said composition is in the form of effervescent tablet.

10. An effervescent formulation composition according to claims 1-9, wherein said composition comprises cefaclor as active agent, at least one effervescent acid, at least one effervescent base and additionally binder, lubricant, sweetener and/or taste regulator, flavoring agent, glidant, diluents, disintegrant, coloring agent, surfactant, anti-foaming agent, humectants, acidic agent and basic agent.

11. An effervescent formulation composition according to claims 1-10, wherein 1-60% of cefaclor, 5-70% of effervescent acid, 5-40% of effervescent base, 1-20% of surfactant, 0.3-6% of binder, 2-10% of lubricant ,0.5-7% of sweetener and/or taste regulator, 1-4% of flavoring agent and 0-3% of coloring agent with respect to the total weight of the unit dosage are used in said composition.

12. An effervescent formulation pharmaceutical composition according to claims 1-11, wherein a second acidic agent as effervescent acid is used in addition to acid anhydrous and this second acidic agent is selected from a group comprising sodium citrate, sodium acetate, dibasic sodium phosphate, tribasic sodium phosphate, monobasic sodium phosphate, sodium acid pyrophosphate and sodium acid sulphite.

13. An effervescent formulation pharmaceutical composition according to claims 1-12, wherein in said composition cefaclor: acid anhydrous ratio is in the range of 2:10 to 7:10 and cefaclor: carbonate anhydrous ratio is in the range of 4:10 to 8:10.

14. An effervescent formulation pharmaceutical composition described in any of the previous claims, wherein said composition further comprise a second active agent, which is selected from beta lactamase inhibitor and cephalosporins, preferably clavulanic acid or a pharmaceutically acceptable form wherein the forms are solvates, hydrates, enantiomers, racemates, organic salts, inorganic salts, polymorphs, crystal and amorphous forms or in free form and/or as a combination of these forms, and even more preferably potassium clavulanate is used as second active agent.

15. A process for the preparation of the pharmaceutical composition according to claims 1-14, wherein said process comprises the steps of obtaining the granulation solution by mixing at least one binder, at least one lubricant and deionized water; sieving effervescent acid, effervescent base and surfactant and granulating them with the granulation solution; adding cefaclor, sweetener and flavoring agent after drying and sieving the granules obtained in the second step.

## Patentansprüche

1. Eine übersprudelnde Formulierung, **dadurch gekennzeichnet, daß** die besagte Zusammensetzung pharmazeutisch aus annehmbaren Mengen von Cefaclor in Hydratform besteht, mindestens eine wasserfreie Säure und wasserfreies Karbonat

2. Eine übersprudelnde Formulierung nach Anspruch 1, wobei Cefaclor in Hydratform in Einheitsdosis im Bereich von 500 mg bis 1500 mg vorhanden ist.

3. Eine übersprudelnde Formulierung nach Ansprüche 1 bis 2, wobei wasserfreie Säure, die als sprudelnde Säure verwendet wird, von wasserfreien organischen Säuren wie wasserfreie Zitronensäure, wasserfreie Weinsäure, wasserfreie Apfelsäure, wasserfreie Fumarsäure, wasserfreie Askorbinsäure, wasserfreie Adipinsäure und wasserfreie Bernsteinsäure ausgewählt ist.

4. Eine übersprudelnde Formulierung nach Anspruch 3, wobei wasserfreie Zitronensäure als wasserfreie Säure verwendet wird.

5. Eine übersprudelnde Formulierungszusammensetzung nach Ansprüche 1 bis 4, wobei wasserfreies Karbonat, das als sprudelnde Base verwendet wird, von wasserfreien Basemitteln wie wasserfreies Natriumkarbonat, wasserfreies Natriumbikarbonat, wasserfreies Kaliumkarbonat, wasserfreies Lysinkarbonat, wasserfreies Argininkarbonat und wasserfreies Calciumkarbonat ausgewählt ist.

6. Eine übersprudelnde Formulierungszusammensetzung nach Anspruch 5, wobei wasserfreies Natriumbikarbonat als wasserfreies Karbonat verwendet wird.

7. Eine übersprudelnde Formulierungszusammensetzung nach Ansprüche 1 bis 6, wobei Cefaclor, die als Agens verwendet wird, in einer der Formen wie Monohydrat, Dihydrat, Trihydrat vorhanden ist.

8. Eine übersprudelnde Formulierungszusammensetzung nach Anspruch 7, wobei Cefaclor, die als Agens verwendet wird, in Form von Monohydrat ist.

9. Eine übersprudelnde Formulierungszusammensetzung nach Ansprüche 1 bis 8, wobei die besagte Zusammensetzung in Form von Brausetablette ist.

10. Eine übersprudelnde Formulierungszusammensetzung nach Ansprüche 1 bis 9, wobei die besagte Zusammensetzung Cefaclor als Agens, mindestens eine sprudelnde Säure, mindestens eine sprudelnde Base und zusätzliche Bindemittel, Schmiermittel, Süßstoffe und/oder Geschmacksregler, Aromastoffe, Fließ-regulierungsmittel, Verdünnungsmittel, Disintegrin, Farbstoffe, Benetzungsmittel, Antischaummittel, Befeuchtungsmittel, Säuremittel und Basemittel beinhaltet.

11. Eine übersprudelnde Formulierungszusammensetzung nach Ansprüche 1 bis 10, wobei 1-60% Cefaclor, 5-70% Brausesäure, 5-40% Brausebase, 1-20% Benetzungsmittel, 0.3-6% Bindemittel, 2-10% Schmiermittel, 0.5-7% Süßstoffe und/oder Geschmacksregler, 1-4% Aromastoffe und 0-3% Farbstoffe mit Bezug auf das Totalgewicht vom Einheitsdosis in besagter Zusammensetzung verwendet wird.

12. Eine übersprudelnde pharmazeutische Formulierungszusammensetzung nach Ansprüche 1 bis 11, wobei eine zweite Säuremittel als Brausesäure zusätzlich zu wasserfreie Säure verwendet wird und diese Säuremittel ist von einer Gruppe ausgewählt, die Natriumcitrat, Natriumacetat, doppelbasische Natriumphosphat, dreibasische Natriumphosphat, monobasische Natriumphosphat, Natriumsäurepyrophosphat und Natriumsäuresulfit beinhaltet.

13. Eine übersprudelnde pharmazeutische Formulierungszusammensetzung nach Ansprüche 1 bis 12, wobei sich in besagter Zusammensetzung Cefaclor: wasserfreie Säure Verhältnis im Bereich von 2:10 zu 7:10 und Cefaclor: wasserfreie Karbonat Verhältnis im Bereich von 4:10 zu 8:10 befindet.

14. Eine übersprudelnde pharmazeutische Formulierungszusammensetzung beschrieben nach den vorhergehenden Ansprüche, wobei besagte Zusammensetzung weiter eine zweite Agens beinhaltet, die von Betalactamase Inhibitor und Cephalosporin ausgewählt wird, bevorzugt sind Clavulansäure oder eine pharmazeutisch zulässige Form, wobei die Formen Solvate, Hydrate, Enantiomere, Racemate, organische Salze, anorganische Salze, Polymorphe, Kristall- und gestaltlose Formen oder freie Form und/oder eine Kombination von diesen Formen sind, und sogar mehr bevorzugt ist Kaliumclavulanate, die als eine zweite Agens verwendet wird.

15. Ein Prozess für die Vorbereitung von pharmazeutischer Zusammensetzung nach Ansprüche 1 bis 14, wobei besagter Prozess folgende Schritte beinhaltet; nämlich Erhaltung einer Granulationslösung durch Einmischung von mindestens einem Bindemittel, mindestens einem Schmiermittel und deionisiertes Wasser; Sieb von sprudelnde Säure, sprudelnde Base und Benetzungsmittel und deren Granulierung mit Granulierungslösung; Zugabe von Cefaclor, Süßstoffe und Aromastoffe nach Trocknung und Sieb von Körnchen, die im zweiten Schritt erhalten sind.

## Revendications

1. Une formulation effervescente **caractérisée en ce que** ladite composition comprend des quantités pharmaceutiquement acceptables de cefaclor sous forme hydratée, au moins un acide anhydre et carbonate anhydre.

2. Une formulation effervescente selon la revendication 1, dans laquelle le cefaclor sous forme d'hydrate est présent dans la dose unitaire dans la plage de 500 mg à 1500 mg.

3. Une formulation effervescente selon les revendications 1 à 2, dans laquelle l'acide anhydre qui est utilisé comme acide effervescent est choisi parmi les acides organiques anhydres tels que l'acide citrique anhydre, l'acide tartrique anhydre, l'acide malique anhydre, l'acide fumarique anhydre, l'acide ascorbique anhydre, l'acide adipique anhydre et l'acide succinique anhydre.

4. Une composition de formulation effervescente selon la revendication 3, dans laquelle l'acide citrique anhydre est utilisé comme acide anhydre.

5. Une composition de formulation effervescente selon les revendications 1 à 4, dans laquelle le carbonate anhydre utilisé comme base effervescente est choisi parmi les agents basiques anhydres tels que le carbonate de sodium anhydre, le bicarbonate de sodium anhydre, le carbonate de potassium anhydre, le carbonate de lysine anhydre, le carbonate d'arginine anhydre et le carbonate de calcium anhydre.

6. Une composition de formulation effervescente selon la revendication 5, dans laquelle le bicarbonate de sodium anhydre est utilisé comme carbonate anhydre.

7. Une composition de formulation effervescente selon les revendications 1 à 6, dans laquelle le cefaclor qui est utilisé comme agent actif est présent sous une forme de monohydrate, de dihydrate, de trihydrate.

8. Une composition de formulation effervescente selon la revendication 7, dans laquelle le cefaclor qui est utilisé comme agent actif est sous forme monohydratée.

9. Une composition de formulation effervescente selon les revendications 1 à 8, dans laquelle ladite composition se présente sous la forme d'un comprimé effervescent.

10. Une composition de formulation effervescente selon les revendications 1 à 9, dans laquelle ladite composition comprend du cefaclor comme agent actif, au moins un acide effervescent, au moins une base effervescente et en outre un liant, un lubrifiant, un édulcorant et/ou un régulateur de goût, un agent aromatisant, un agent de glissement, des diluants, en désintégrant, un agent colorant, un tensioactif, un agent antimousse, des humectants, un agent acide et un agent de base.

11. Une composition de formulation effervescente selon les revendications 1 à 10, dans laquelle 1 à 60% de cefaclor, 5-70% d'acide effervescent, 5-40% de base effervescente, 1 à 20% de tensioactif, 0,3 à 6% de liant, 2-10% de lubrifiant, 0,5 à 7% d'édulcorant et/ou régulateur de goût, 1 à 4% d'agent aromatisant et 0 à 3% d'agent colorant par rapport au poids total de la dose unitaire sont utilisés dans ladite composition.

12. Une composition pharmaceutique de formulation effervescente selon les revendications 1 à 11, dans lequel un deuxième agent acide comme acide effervescent est utilisé en plus de l'acide anhydre et ce deuxième agent acide est choisi parmi un groupe comprenant du citrate de sodium, l'acétate de sodium, le phosphate de sodium dibasique, le phosphate de sodium tribasique, le phosphate de sodium monobasique, le pyrophosphate d'acide sodique et le sulfite acide de sodium.

13. Une composition pharmaceutique de formulation effervescente selon les revendications 1 à 12, dans laquelle dans la composition cefaclor: le rapport d'acide anhydre est dans la plage de 2: 10 à 7:10 et le rapport de cefaclor: carbonate anhydre est dansla plage de 4: 10 et 8: 10.

14. Une composition pharmaceutique de formulation effervescente selon l'une quelconque des revendications précédentes, dans laquelle ladite composition comprend en outre un deuxième agent actif, qui est choisi parmi l'inhibiteur de la bêta-lactamase et les céphalosporines, de préférence l'acide clavulanique ou une forme pharmaceutiquement acceptable dans laquelle les formes sont des solvates, des hydrates, des énantiomères , des racémates, des sels organiques, des sels inorganiques, des polymorphes, des cristaux et des formes amorphes ou sous forme libre et / ou en combinaison de ces formes, et encore plus préférentiellement le clavulanate de potassium est utilisé comme deuxième agent actif.

15. Procédé de préparation de la composition pharmaceutique selon les revendications 1 à 14, dans lequel ledit procédé comprend les étapes consistant à obtenir la solution de granulation en mélangeant au moins un liant, au moins un lubrifiant et de l'eau désionisée; tamiser l'acide effervescent, la base effervescente et le tensioactif et les granuler avec la solution de granulation; ajouter du cefaclor, un édulcorant et un agent aromatisant après séchage et tamisage des granulés obtenus dans la deuxième étape.
